# EUROPEAN PATENT SPECIFICATION

(11) **EP 1 827 329 B1**
(45) Date of publication and mention of the grant of the patent: **23.11.2016**
(21) Application number: 05786761.6
(22) Date of filing: 05.10.2005
(51) Int. Cl.: A61F 9/008

(54) **PROTECTIVE DEVICE FOR OPHTHALMIC LASER TREATMENT**
SCHUTZVORRICHTUNG FÜR AUGENLASERBEHANDLUNG
DISPOSITIF DE PROTECTION POUR TRAITEMENT AU LASER OPHTALMIQUE

(30) Priority: 02.12.2004 US 883
(43) Date of publication of application: 05.09.2007
(73) Proprietor: SIE AG, Surgical Instrument Engineering, 2562 Port (CH)
(72) Inventor: RATHJEN, Christian, 28197 Bremen (DE)
(74) Representative: Rentsch Partner AG
(86) International application number: PCT/CH2005/000578
(87) International publication number: WO 2006/058443

(56) References cited:
- WO-A-98/49955
- WO-A-03/041623
- US-A- 5 318 780
- US-A- 5 437 658
- US-A- 5 779 696

## Description

### Field of the Invention

The present invention relates to a protective device for ophthalmic laser treatment. Specifically, the present invention relates to a protective device for protecting an eye from direct contact by a reference body disposed between a laser applicator and the eye.

### Background of the Invention

Laser technology has been used in ophthalmic surgery for many years. Significant progress in laser technology has made it possible to also use laser technology for cutting an exterior layer of the cornea, a procedure that used to be performed before by means of a microkeratome using a mechanical blade. For cutting tissue inside the cornea, laser producing ultra-short laser pulses are used, for example pico second lasers producing pulse widths of 1 ps to 10ps (1ps=10⁻¹²s) or femto second lasers producing pulse widths of typically 1fs to 1000fs (1fs=10⁻¹⁵s). The required high intensities to dissolve tissue are achieved by focusing strongly the laser beam to a few microns. For cutting, it is necessary to place precisely pulses next to each other. For ophthalmic laser surgery to achieve sufficient precision, there needs to be a precise and stable coupling of the laser applicator to the operative area of the patient's eye.

In U.S. Pat. No. 6,730,074, a system is described for accurately guiding a laser focal point along a predetermined path within the stroma of a cornea. The system includes a contact lens used as a reference body for the laser applicator. The contact lens is mounted in a suction ring and pressed gently against the exterior surface of the cornea. Applying a vacuum or a partial vacuum to the suction ring, the contact lens is held against the cornea. For example, the suction ring is fixed to the laser applicator.

In U.S. Pub. No. 2002/0103481, described is a stabilization and applanation device for reconfiguring the cornea of an eye for opthalmic laser surgery. The device includes an applanation lens used as a reference body for the laser applicator. A cone including the lens is connected to the laser applicator. A suction ring is attached to the eye and the cone is coupled to the suction ring. Thereby, the laser applicator is positioned in a defined relationship with the surface of the patient's eye applanated by the lens.

The reference bodies used in the prior art are in direct contact with the cornea of the patient's eye. Consequently, there is a danger of transferring infectious material, such as viruses, from one patient to another patient, if the same reference bodies are re-used without proper sterilization. Harm to the patient's eye may also be caused by abrasion, resulting from sliding movements, or by exposure to materials that are non-biocompatible. Using only disposable devices for contacting directly the patient's eye, as proposed in U.S. Pub. No. 2002/0103481, would help to prevent some of these problems. However, the reference bodies are precision devices and disposing thereof after one-time use causes a significant financial cost, to be covered by the patient or his medical insurance.

There are other risks and dangers to the patient's eye from having reference bodies in direct contact with the patient's eye, which cannot be prevented by limiting usage of reference bodies to one-time use. For example, when the reference body is in direct contact with the patient's eye, there is a risk that the patient's eye is being injured by sharp edges of a broken reference body. Injuries to the patient's eye may also result from decomposition products of the reference body or deformations of the reference body, produced when the laser focal point is moved accidentally or deliberately into the area of the reference body.

U.S. Pat. No. 5,318,780 describes a strong transparent film resembling a thin, soft hydrophilic corneal contact lens which could be used as a protective corneal mask or as an ablatable mask useful in laser keratectomy.

U.S. Pat. No. 5,437,658 describes a thermokeratoplasty system for modifying corneal curvature comprising a radiation distributing device for introducing electromagnetic radiation to a multiplicity of locations on the surface of the cornea and a positioning structure for positioning the radiation distributing device in close, fixed relationship to the cornea. The positing structure comprises a thornton ring and a rigid dome. According to 5,437,658, a prophylactic membrane is placed on the cornea and held in place by small protrusions of the thornton ring. The protrusions penetrate the prophylactic membrane and the corneal surface to hold the thornton ring in place during the laser thermokeratoplasty. The rigid dome is held on the thornton ring by creating a vacuum in between the two members or using a snap-on connector.

### Summary of the Invention

It is an object of this invention to provide a protective device for ophthalmic laser treatment, the protective device protecting an eye from direct contact by a reference body disposed between a laser applicator and the eye. Particularly, is an object of this invention to provide a protective device for ophthalmic laser treatment wherein a transparent reference body is disposed between a laser applicator and a patient's eye and wherein the eye is treated by means of a laser, producing ultra-short laser pulses, for example. Particularly, the protective device for ophthalmic laser surgery should prevent the transfer from patient to patient of infectious material attached to reference bodies, without having to limit usage of a reference body to one-time. Moreover, the protective device for ophthalmic laser surgery should prevent injuries to the eye from broken or deformed reference bodies.

According to the present invention, these aims are achieved in particular by the elements of the independent claims. Further advantageous embodiments also follow from the dependent claims and the description.

According to the present invention, the above-mentioned objects are particularly achieved in that a protective device for ophthalmic laser treatment comprises a transparent foil, for protecting an eye from direct contact by a reference body disposed between a laser applicator and the eye. The reference body is also referred to as "contact body" or "applanation body". For example, the laser is a femto second laser or a pico second laser producing ultra-short laser pulses with typical widths of 1 ps to 10ps or 1fs to 1000fs, respectively. Preferably, the foil is disposable. When the reference body is applied onto the eye, the foil is protecting the eye from direct contact by the reference body. The reference body is coupled removably to the laser applicator or the reference body is fixed permanently to the laser applicator. When the laser is activated, the laser pulses are projected from the laser applicator through the reference body and the foil to dissolve tissue of the eye, particularly corneal tissue. In addition to protecting the eye from direct contact with the reference body, disposing a foil between the reference body and the eye has the advantage that the focal point of the laser cannot only be adjusted to positions within cornea tissue of the eye, but also to positions within the foil to make open cuts in the cornea, without damaging the reference body. Moreover, disposing a foil between the reference body and the eye has the advantage that this makes it possible to choose freely the material used for the reference body.

According to the invention, the protective device further comprises means for disposing the foil between the reference body and the eye. The means for disposing the foil facilitate the handling of the foil as well as the placement and/or positioning of the foil.

In an embodiment, the means for disposing the foil include a carrier frame and the foil is attached to the carrier frame. For example, the carrier frame and the foil are made in one piece. A carrier frame makes it easier for a user to handle the foil and to place the foil. The carrier frame also makes it possible to package and unpackage easily the foil with or from a protective packaging that keeps the foil sterile.

According to the invention, the means for disposing the foil further include a suction ring designed for attachment to the eye around an operative area. The suction ring is designed to receive the foil such that the operative area is covered by the foil in a state of the suction ring being attached to the eye. For example, the foil is attached to the suction ring such that the operative area is covered by the foil in a state of the suction ring being attached to the eye. Preferably, however, the suction ring is designed to receive the carrier frame such that the operative area is covered by the foil in a state of the suction ring being attached to the eye. For example, the carrier frame and/or the suction ring are provided with coupling means for removably coupling the carrier frame to the suction ring. In an alternative embodiment, the carrier frame (and/or to the reference body or the laser applicator) is provided with coupling means for removably coupling the carrier frame to the reference body or the laser applicator, the laser applicator having the reference body attached thereto. According to the invention, the suction ring is provided with coupling means for removably coupling the suction ring to the reference body or the laser applicator, e.g. the laser applicator having the reference body attached thereto, such that the foil is interposed between the eye and the reference body in the state of the suction ring being attached to the eye.

In a preferred embodiment, the suction ring and the foil form a vacuum chamber in the state of the suction ring being attached to the eye. In an embodiment, the suction ring has a first opening closed off by the foil, the suction ring has a second opening closed off by the eye, in the state of the suction ring being attached to the eye, and the operative area is in contact with the foil in the state of the suction ring being attached to the eye. In a further embodiment, the first opening is confined by a protrusion of the suction ring extending to an interior of the suction ring and the first opening is closed off by the foil placed on the protrusion. For example, the protrusion has a slanted edge for contacting the eye in the state of the suction ring being attached to the eye.

In a further preferred embodiment, the means for disposing the foil are configured to position the foil such that there is a gap between the foil and the reference body, in a state prior to the reference body being applied to the eye, and such that, in a process of the reference body being applied to the eye, a contact region of the foil, that comes into contact with the reference body, is interposed between the reference body and the eye, and fluid (e.g. air) which is located in the area between the contact region and the reference body is forced out of the latter area. Depending on the embodiment, the gap between the foil and the reference body is defined by the design of the carrier frame and/or the suction ring, keeping the foil at a distance from the reference body. With increasing applanation of the eye by the reference body, the contact region of the foil that comes into contact with the reference body increases in size from a first point of contact. As a result, inclusions of fluid, e.g. air, between the contact region and the reference body are forced out in the increasingly enlarging contact region, extending out from the initial point of contact, so that no fluid (air) pockets remain. The forcing of fluid (air) out of the area between the contact region and the reference body has the advantage that the transmission of laser pulses from the laser applicator through the reference body and the foil into the eye is not influenced negatively by inclusions of fluid (air), i.e. the laser pulses are not refracted or diffused by inclusions of fluid (air). In the process of the foil being disposed between the reference body and the eye, the gap between the foil and the reference body prevents the foil from being damaged when the foil is moved relative to the reference body while in contact with the reference body. For example, scratching of the foil is prevented when a translatory or rotary motion is applied to the foil, e.g. through a bayonet coupling used for attaching the carrier frame and/or the suction ring with the foil to the laser applicator.

In an embodiment, the foil is adhesive on at least one side such that the foil attaches removably to the reference body at least in a contact region of the foil in a state of the foil being interposed between the reference body and the eye. Particularly, in combination with the embodiment that supports forcing out of air located in the area between the contact region and the reference body, an adhesive foil makes possible a stable fixation of the foil to the reference body without inclusions of air.

In various alternative or complementing embodiments, the foil has a specified area of non-transparency to thereby limit an operative area of the eye, the foil has a refraction index corresponding to the refraction index of the tissue of the eye and/or the refraction index of the reference body, the foil has surface structures on an interior surface of the foil contacting the eye to thereby conduct fluids from and to an exterior surface of the eye being contacted by the foil, the foil has elastic coating on an interior surface of the foil contacting the eye to thereby even out uneven areas of an exterior surface of the eye being contacted by the foil, the foil has perforations to make possible the exchange of fluids between an interior surface of the foil contacting the eye and an exterior surface of the foil contacting the reference body, the foil is made of polyvinyl chloride, polyethylene or polypropylene, the foil is made of medical grade plastic, and/or the foil is selected with a specified thickness to determine an operative depth.

### Brief Description of the Drawings

The present invention will be explained in more detail, by way of example, with reference to the drawings in which:
Figure 1 a shows a schematic cross section of the cornea of a patient's eye wherein a suction ring, having a transparent foil fixed thereon, is attached to the eye.
Figure 1b shows the schematic cross section of the cornea of Figure 1 a wherein a reference body, fixed to a laser applicator, is coupled to the suction ring such that the transparent foil is interposed between the reference body and the eye.
Figure 2a shows a schematic cross section of the cornea of a patient's eye wherein a suction ring, having a transparent foil placed therein, is attached to the eye.
Figure 2b shows the schematic cross section of the cornea of Figure 2a wherein a reference body, fixed to a laser applicator, is coupled to the suction ring such that the transparent foil is interposed between the reference body and the eye.
Figure 3a shows a schematic cross section of the cornea of a patient's eye wherein a transparent foil is placed directly onto the eye and a suction ring, attached to the eye, stabilizes the position of the foil relative to the eye.
Figure 3b shows the schematic cross section of the cornea of Figure 3a wherein a reference body, fixed to a laser applicator, is coupled to the suction ring such that the transparent foil is interposed between the reference body and the eye.
Figure 4a shows a schematic cross section of the cornea of a patient's eye wherein a suction ring, having a transparent foil placed therein, is attached to the eye, and wherein a reference body is held in the suction ring such that the transparent foil is interposed between the reference body and the eye
Figure 4b shows the schematic cross section of the cornea of Figure 4a wherein a laser applicator is coupled to the suction ring and to the reference body.
Figure 5a shows a schematic cross section of the cornea of a patient's eye, having a suction ring attached thereto, and a laser applicator with a reference body covered by a transparent foil attached to the laser applicator.
Figure 5b shows the schematic cross section of the cornea of Figure 5a wherein the reference body and the laser applicator are coupled to the suction ring such that the transparent foil is interposed between the reference body and the eye.
Figure 6a shows a schematic cross section of the cornea of a patient's eye, having a suction ring attached thereto, and a laser applicator, having a reference body with a transparent foil fixed thereon.
Figure 6b shows the schematic cross section of the cornea of Figure 6a wherein the reference body and the laser applicator are coupled to the suction ring such that the transparent foil is interposed between the reference body and the eye.
Figure 7a shows a schematic cross section of the cornea of a patient's eye and of a suction ring, having a transparent foil fixed thereon, coupled to the laser applicator.
Figure 7b shows the schematic cross section of the cornea of Figure 7a wherein the suction ring, coupled to the laser applicator, is attached to the eye such that the transparent foil is interposed between the reference body and the eye.
Figure 8a shows a schematic cross section of the cornea of a patient's eye and of a suction ring, having a transparent foil placed thereon, coupled to the laser applicator.
Figure 8b shows the schematic cross section of the cornea of Figure 8a wherein the suction ring, coupled to the laser applicator, is being applied to the eye such that the transparent foil is interposed between the reference body and the eye.
Figure 8c shows the schematic cross section of the cornea of Figure 8a wherein the suction ring, coupled to the laser applicator, is attached to the eye such that the transparent foil is interposed between the reference body and the eye.
Figure 9 shows a cross section of a protective device for ophthalmic laser treatment, having a suction ring with a transparent foil placed therein.
Figure 10 shows a schematic cross section of the cornea of a patient's eye, having a transparent foil placed thereon, wherein different positions of the focal point of a pulsed laser beam, projected through a reference body and the foil, are illustrated.

### Detailed Description of the Preferred Embodiments

The reference numeral 1 refers to a patient's eye and the reference numeral 11 refers to the cornea of the eye 1.

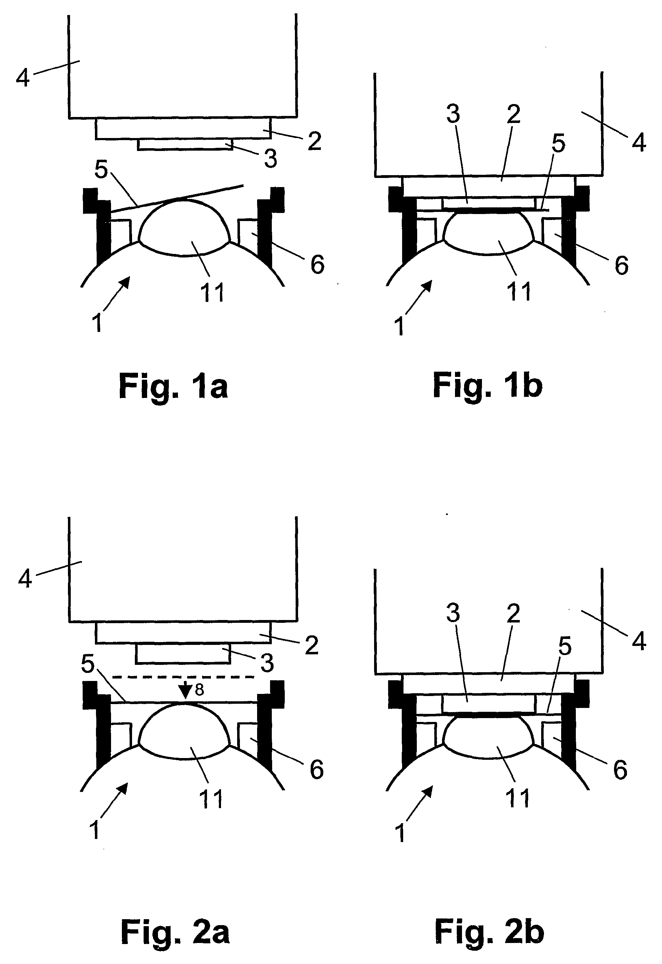

The reference numeral 4 refers to a laser applicator. Preferably, the laser applicator 4 is configured to project pulsed and focused laser beams having ultra-short laser pulses. For example, the laser applicator 4 is configured to project pulse widths of 1 ps to 10ps (pico second laser) or pulse widths of typically 1 fs to 1000fs (femto second laser). The laser is preferably integrated in the laser applicator 4. However, it is also possible to have the laser external to the laser applicator 4, coupled through optical links, e.g. fibers. As is illustrated in Figures 1a, 1b, 2a, 2b, 3a, 3b, 4a, 4b, 5a, 5b, 6a, 6b, 7a, 7b, 8a, 8b, and 8c the laser applicator 4 includes an applicator end piece 2.

As is illustrated in Figures 1a, 1b, 2a, 2b, 3a, 3b, 5a, 5b, 6a, 6b, 7a, 7b, 8a, 8b, 8c, 9 and 10, a reference body 3 is attached to the laser applicator 4, e.g. to the applicator end piece 2. The reference body 3 is either permanently fixed to the laser applicator 4 or coupled removably to the laser applicator 4, for example by means of a snap or screw mechanism. The reference body 3 is transparent and preferably made from clear (optical) glass or polymers. The reference body 3 may have a cylindrical shape or the shape of a contact lens, for example.

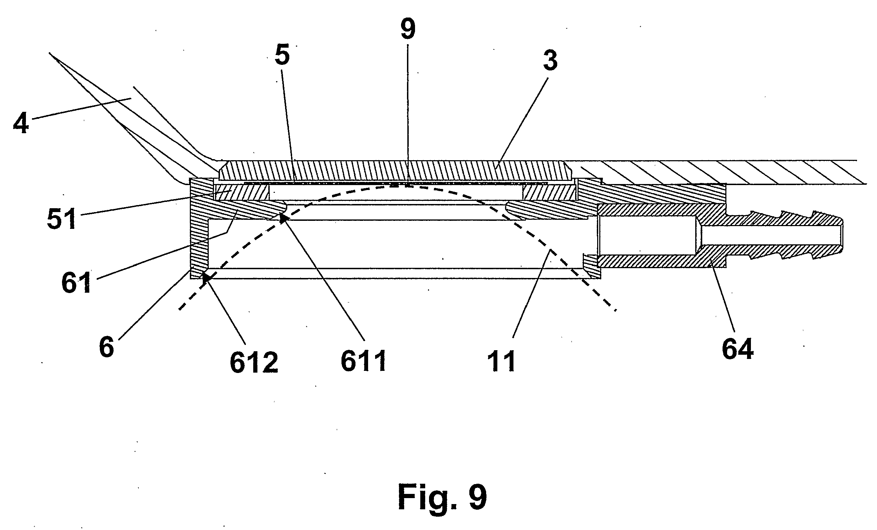

The reference numeral 6 refers to a suction ring, which is held in place on the patient's eye 1 by means of a vacuum or a partial vacuum generated by a vacuum pump (not illustrated). The suction ring 6 and the laser applicator 4 (or the applicator end piece 2, respectively) are configured for removably interconnecting with each other, to thereby stabilize the position of the pulsed laser beam relative to the patient's eye. In Figure 9, the suction ring 6 is illustrated having a tube coupling 64 for connecting the suction ring 6 to a vacuum pump through a tube.

The reference numeral 5 refers to a foil, which is transparent at least in some areas so that a pulsed laser beam can be projected through the foil 5 in those areas. The foil 5 is flexible and has a thickness of less than one millimeter. For embodiments where the foil 5 adheres flexibly to the reference body 3, the thickness of the foil 5 is preferably not thicker than 200µm. For embodiments where the foil 5 is rather stiff and does not need to adhere flexibly to the reference body 3, the thickness of the foil 5 can be thicker than 200µm. Preferably, the foil 5 is provided in different defined thickness so that a specific foil 5, interposed between reference body 3 and the eye 1, can be selected to adjust the position of the focal point of the pulsed laser beam to thereby determine the operative depth. Preferably, the foil 5 is made from medical grade plastic and has a refraction index corresponding to the refraction index of the tissue of the eye 1 and/or the refraction index of the reference body 3. For example, the foil is made of polyvinyl chloride, polyethylene or polypropylene. In an embodiment, the foil is provided with perforations to make possible the exchange of fluids between the interior surface of the foil 5 contacting the eye 1 and the exterior surface of the foil 5 contacting the reference body 3. In a further embodiment, the foil 5 is provided with surface structures on the interior surface to make it possible to conduct fluids from and to the exterior surface of the eye 1 being contacted by the foil 5. In a further embodiment, the foil 5 is provided with an elastic coating on the interior surface of the foil 5 contacting the eye 1 to make it possible to even out uneven areas, for example scars, of the exterior surface of the eye 1. In yet a further embodiment, the foil 5 has at least one adhesive side for attaching the foil 5 to the reference body 3.

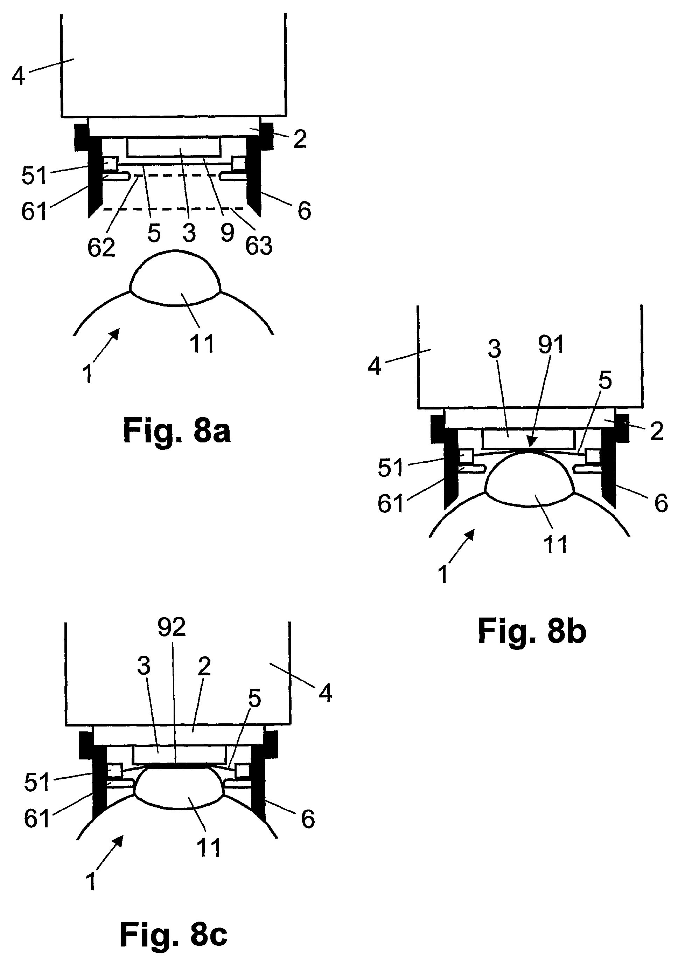

As is illustrated schematically in Figures 8a, 8b, 8c and 9, preferably, the foil 5 is attached to a carrier frame 51. For example, the carrier frame 51 and the foil 5 are made in one piece. Preferably, the suction ring 6 is configured to receive the carrier frame 51 with the foil 5 attached thereto. In alternative variants, the carrier frame 51 is provided with coupling means for removably coupling the carrier frame 51 with the foil 5 to the suction ring 6, or the carrier frame 51 is provided with coupling means for removably coupling the carrier frame 51 with the foil 5 to the reference body 3, the laser applicator 4, or the applicator end piece 2.

In the following paragraphs, different embodiments of a protective device for ophthalmic surgery, each device having at least a sterile foil 5, and of disposing the foil 5 between the reference body 3 and an operative area of the eye 1 are described with reference to Figures 1a, 1b, 2a, 2b, 3a, 3b, 4a, 4b, 5a, 5b, 6a, 6b, 7a, and 7b. In the examples illustrated, the operative area is part of the cornea 11.

In the embodiment illustrated in Figure 1a, the protective device includes a suction ring 6 having the transparent foil 5 attached thereon. As illustrated schematically in Figure 1a, the transparent foil 5 may be attached to the suction ring 6 such that parts of the foil 5 are loose and thus maneuverable. At first, the suction ring 6 with the transparent foil 5 attached thereon, is placed around the operative area of the eye 1 such that the foil 5 covers the operative area. The foil 5 (with or without the carrier frame 51) is either fixed permanently to the suction ring 3 (e.g. by the manufacturer) or is attached removably to the suction ring 6, for example through adhesion, press fit, or by fastening means such as a clamping mechanism. Thereafter, the suction ring 6 is attached to the eye 1. As is illustrated in Figure 1b, the protective device is provided with coupling means for coupling the laser applicator 4 (or the applicator end piece 2, respectively) to the suction ring 6. The reference body 3 is applied onto the eye 1 in that the laser applicator 4, with the reference body 3 attached thereon, is coupled to the suction ring 6. Through coupling the laser applicator 4 to the suction ring 6, the foil 5 is interposed between the reference body 3 and the eye 1.

In the embodiment illustrated in Figure 2a, the protective device includes a suction ring 6 configured to receive the transparent foil 5. At first, the suction ring 6 is placed around the operative area of the eye 1 and attached to the eye 1. Then, as is indicated schematically by arrow 8, the foil 5 (with or without the carrier frame 51) is placed into the opening of the suction ring 6 such that the foil 5 covers the operative area. Thereafter, as illustrated in Figure 2b, the laser applicator 4, with the reference body 3 attached thereon, is coupled to the suction ring 6. Thereby, the foil 5 is interposed between the reference body 3 and the eye 1.

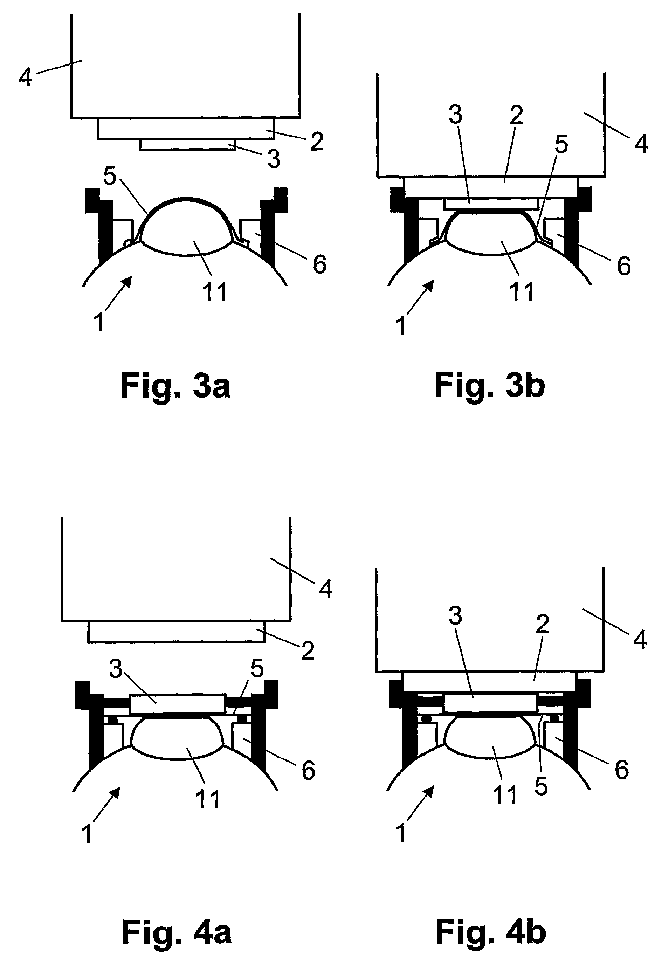

In the embodiment illustrated in Figure 3a, first, the transparent foil 5 is placed directly onto the eye 1 to cover the operative area. Thereafter, the suction ring 6 is placed onto the eye 1 such that foil 5 is stabilized relative to the operative area by the suction ring 6. Finally, as illustrated in Figure 3b, the laser applicator 4, with the reference body 3 attached thereon, is coupled to the suction ring 6. Thereby, the foil 5 is interposed between the reference body 3 and the eye 1.

In the embodiment illustrated in Figure 4a, the protective device includes a suction ring 6 configured to receive the transparent foil 5 and to attach the reference body 3 to the suction ring 6. The suction ring 6, having the transparent foil 5 placed therein and the reference body 3 attached thereto, is placed around the operative area and attached to the eye 1. The foil 5 (with or without the carrier frame 51) is placed into the suction ring 6, before the reference body 3 is attached to the suction ring 6. The foil 5 is either fixed permanently to the suction ring 6, for example by the manufacturer, or placed removably into the suction ring 6. The reference body 3 is coupled removably to the suction ring 6, for example by means of a snap or screw mechanism. When attaching the suction ring 6 to the eye 1, in this embodiment, the foil 5 is interposed between the reference body 3 and the eye 1. Finally, as illustrated in Figure 4b, the laser applicator 4 is coupled to the suction ring 6 and to the reference body 3. In an alternative embodiment, the reference body 3 is fixed permanently to the suction ring 6 or manufactured with the suction ring 6 as one piece.

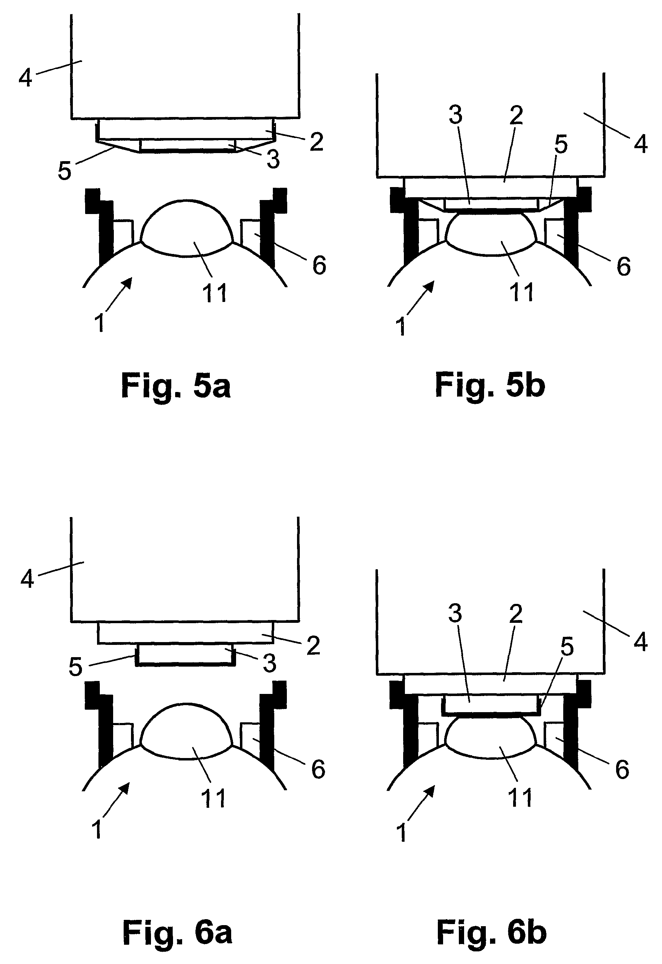

In the scenario illustrated in Figure 5a as background information, the foil 5 (with or without the carrier frame 51) is attached to the laser applicator 4 (or to the applicator end piece 2, respectively) such that the foil 5 covers the part of the reference body 3 to be applied onto the eye 1. The foil 5 is attached removably to the laser applicator 4, for example through adhesion, vacuum, press fit, or by fastening means such as a clamping mechanism. After having placed the suction ring 6 around the operative area and attached to the eye 1, the laser applicator 4, with the covered reference body 3 attached thereto, is coupled to the suction ring 6. Thereby, the foil 5 is interposed between the reference body 3 and the eye 1, as illustrated in Figure 5b.

In the scenario illustrated in Figure 6a as background information, the foil 5 (with or without the carrier frame 51) is attached to the reference body 3 attached to the laser applicator 4 (or to the applicator end piece 2, respectively) such that the foil 5 covers the part of the reference body 3 to be applied onto the eye 1. The foil 5 is attached removably to the reference body 3 through adhesion, press fit, or by fastening means such as a clamping mechanism. After having placed the suction ring 6 around the operative area and attached to the eye 1, the laser applicator 4, with the covered reference body 3 attached thereto, is coupled to the suction ring 6. Thereby, the foil 5 is interposed between the reference body 3 and the eye 1, as illustrated in Figure 6b.

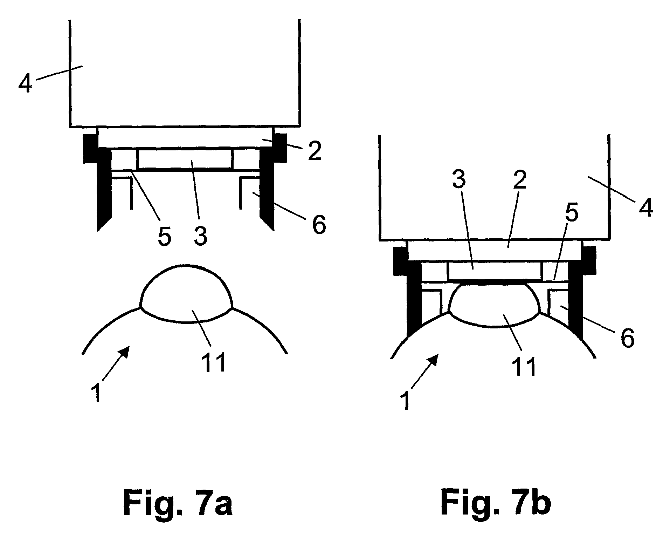

In the embodiment illustrated in Figure 7a, the protective device includes a suction ring 6 and a foil 5 configured to be coupled removably to the laser applicator 4. The suction ring 6 and the foil 5 are coupled removably to the laser applicator 4 prior to attaching the suction ring 6 to the eye 1, as shown in Figure 7b. Attaching the suction ring 6 to the eye 1 after having coupled the suction ring 6 to the laser applicator 4 and the reference body 3 is applicable also to the embodiments illustrated in Figures 1a, 1b, 2a, 2b, 3a, 3b, 4a, and 4b.

In the preferred embodiment, illustrated in Figures 8a, 8b, 8c, and 9, the protective device includes a suction ring 6 configured to receive the foil 5. Preferably, the suction ring 6 is configured to receive the carrier frame 51 having the foil 5 attached thereto. For example, the suction ring 6 has a protrusion 61 extending to the interior of the suction ring 6. Preferably, the protrusion 61 extends fully along the inside wall of the suction ring and encloses an operative opening 62. Partial protrusions, that do not encircle fully the operative opening 62, are also possible. The foil 5 (with or without a carrier frame 51) is positioned fixed or removably on the protrusions 51 thereby closing off the operative opening 62. For a removable positioning of the foil 5, the suction ring 6 and/or the carrier frame 51 are provided with coupling means, for example a snapping mechanism, or are designed for a press fit. The suction ring 6 and/or the laser applicator 4 are provided with coupling means for connecting the protective device removably to the laser applicator 4, for example a snapping mechanism, a screwing mechanism, or a bayonet coupling. For example, the coupling means are part of the reference body 3 or the applicator end piece 2. As can be seen in Figure 8a, the suction ring 6 is configured for placement of the foil 5 (with or without the carrier frame 51) in the suction ring 6 such that there is an (air) gap 9 between the foil 5 and the reference body 3 when the suction ring 6 is coupled to the laser applicator 4. When the suction ring 6 is applied to the eye 1, the cornea 11 first enters the suction ring 6 through the application opening 63 and then through the operative opening 62. As is illustrated in Figure 8b, the foil 5 is pressed against the reference body 3 by the eye 1 when the suction ring 6, with the foil 5 placed therein and the laser applicator attached 4 thereto, is applied onto the eye 1. First the foil comes in contact with the reference body 3 in an initial contact point 91. As the suction ring 6 is applied with more pressure to the eye 1 or cornea 11, respectively, the contact point 91 increases to an expanding contact region. As the contact region expands, any air inclusions between the foil 5 and the reference body 3 are forced out of the contact region. In the same way, air inclusions between the cornea 11 and the foil are forced out of the contact area. Subsequently, as illustrated in the Figure 8c, having the foil 5 interposed between the cornea 11 and the reference body 3, the cornea 11 is brought in a defined state by the reference body 3, for example in an applanation state, if the reference body 3 is plane, and any remaining air is sucked off by the vacuum pump through the tube coupling 64. As can be seen in Figure 9, the protrusion has a slanted edge 611, adapted to the shape of the cornea 11, for contacting the eye 1 without causing injury to its tissue. Also the side walls of the suction ring 6 have on their inside a slanted edge 612 surrounding the application opening 62 and adapted to the shape of the eye 1, e.g. the shape of the cornea 11 or sclera, for contacting the eye 1 without causing injury to its tissue. In a variant, the protrusion 61 is configured to not contact the eye 1 such that air remaining in the area between the eye 1 and the foil 5 is sucked off by the vacuum pump through gaps between the protrusion 61 and the eye 1.

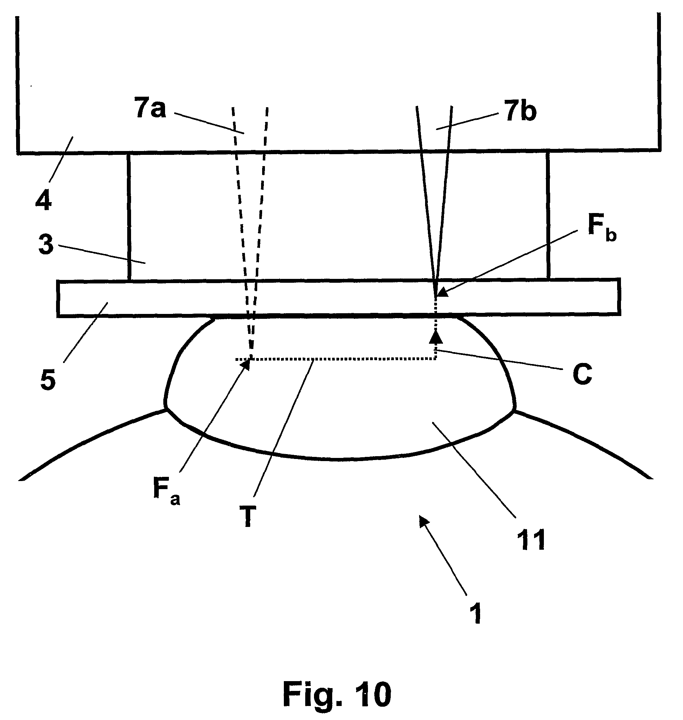

When the laser is activated for surgical treatment of the patient's eye 1, the laser pulses are projected from the laser applicator 4 through the reference body 3 and the foil 5. For example, if the focal point Fₐ of the pulsed laser beam 7a is located at a position within the cornea 11, as illustrated in Figure 10, the laser pulses dissolve tissue of the cornea 11. To make a cut within the cornea, the focal point of the pulsed laser beam is moved along the line T, for example. To make an open cut, the focal point F_{b} of the pulsed laser beam 7b is moved along the line C into the foil 5, thereby not damaging the reference body 3.

It must be pointed out that the configurations of the suction ring 6, the foil 5, the laser applicator 4, and the reference body 3 are illustrated schematically only and that alternative configurations of these components are possible without deviating from the scope of the invention. Particularly, one skilled in the art will understand that specific configurations and features of the protective device illustrated and described with reference to Figures 1a, 1b, 2a, 2b, 3a, 3b, 4a, 4b, 5a, 5b, 6a, 6b, 7a, 7b, 8a, 8b, 8c, and 9 are not mutually exclusive and can be combined with each other. For example, different ways of attaching the foil 5 as described with reference to Figures 1a, 1b, 2a, 2b, 3a, 3b, 4a, 4b, 5a, 5b, 6a, 6b, 7a, and 7b are also applicable to the configuration of the suction ring 6 as described with reference to Figures 8a, 8b, 8c, and 9. Correspondingly, different mechanisms for removably coupling the reference body 3 to the suction ring 6, as described with reference to Figure 4a, can also be combined with the application of the suction ring 6 and the transparent foil 5 as described with reference to Figures 1a, 2a, 2b, 3a, 3b, 7a, 7b, 8a, 8b, 8c, and 9, for example. In its simplest form, the protective device comprises a transparent foil 5 for protecting the eye 1 from direct contact by the reference body 3 disposed between the laser applicator 4 and the eye 1.

## Claims

1. A protective device for ophthalmic laser treatment, comprising a transparent foil (5) for protecting an eye (1) from direct contact by a reference body (3) disposed between a laser applicator (4) and the eye (1), and means for disposing the foil (5) between the reference body (3) and the eye (1), **characterized**
**in that** the means for disposing the foil (5) include a suction ring (6) designed for attachment to the eye (1) around an operative area, in that the suction ring (6) is designed to receive the foil (5) such that the operative area is covered by the foil (5) in a state of the suction ring (6) being attached to the eye (1), and in that the suction ring (6) is provided with coupling means for removably coupling the suction ring (6) to one of the reference body (3) and the laser applicator (4) with the reference body (3) attached thereto, such that the foil (5) is interposed between the eye (1) and the reference body (3) in the state of the suction ring (6) being attached to the eye (1) and being coupled to the reference body (3) or the laser applicator (4) with the reference body (3) attached thereto.

2. Device according to claim 1, **characterized in that** the means for disposing the foil (5) include a carrier frame (51); and **in that** the foil (5) is attached to the carrier frame (51).

3. Device according to claim 2, **characterized in that** the carrier frame (51) is provided with coupling means for removably coupling the carrier frame (51) to the suction ring (6).

4. Device according to one of claims 2 or 3, **characterized in that** the suction ring (6) is designed to receive the carrier frame (51) such that the operative area is covered by the foil (5) in the state of the suction ring (6) being attached to the eye (1).

5. Device according to claim 1, **characterized in that** the foil (5) is attached to the suction ring (6) such that the operative area is covered by the foil (5) in the state of the suction ring (6) being attached to the eye (1).

6. Device according to one of claims 1 to 5, **characterized in that** the suction ring (6) and the foil (5) form a vacuum chamber in the state of the suction ring (6) being attached to the eye (1).

7. Device according to one of claims 1 to 6, **characterized in that** the suction ring (6) has a first opening (62) closed off by the foil (5), **in that** the suction ring (6) has a second opening (63) closed off by the eye (1), in the state of the suction ring (6) being attached to the eye (1), and **in that** the operative area is in contact with the foil (5) in the state of the suction ring (6) being attached to the eye (1).

8. Device according to claim 7, **characterized in that** the first opening (62) is confined by a protrusion (61) of the suction ring (6) extending to an interior of the suction ring (6), and **in that** the first opening (62) is closed off by the foil (5) placed on the protrusion (61).

9. Device according to one of the claims 2 to 4, **characterized in that** the carrier frame (51) and the foil (5) are made in one piece.

10. Device according to one of the claims 1 to 9, **characterized in that** the means for disposing the foil (5) are configured to position the foil (5) such that there is a gap between the foil (5) and the reference body (3) in a state prior to the reference body (3) being applied to the eye (1), and such that in a process of the reference body (3) being applied to the eye (1) a contact region of the foil (5) that comes into contact with the reference body (3) is interposed between the reference body (3) and the eye (1) and fluid which is located in an area between the contact region and the reference body (3) is forced out of the latter area.

11. Device according to one of the claims 1 to 10, **characterized in that** the foil (5) is provided with a specified area of non-transparency to thereby limit an operative area of the eye (1).

12. Device according to one of the claims 1 to 11, **characterized in that** the foil (5) has a refraction index corresponding to the refraction index of the tissue of the eye (1) and/or the refraction index of the reference body (3).

13. Device according to one of the claims 1 to 12, **characterized in that** the foil (5) has surface structures on an interior surface of the foil (5) contacting the eye (1) to thereby conduct fluids from and to an exterior surface of the eye (1) being contacted by the foil (5).

14. Device according to one of the claims 1 to 13, **characterized in that** the foil (5) has elastic coating on an interior surface of the foil (5) contacting the eye (1) to thereby even out uneven areas of an exterior surface of the eye (1) being contacted by the foil (5).

15. Device according to one of the claims 1 to 14, **characterized in that** the foil (5) has perforations to make possible the exchange of fluids between an interior surface of the foil (5) contacting the eye (1) and an exterior surface of the foil (5) contacting the reference body (3).

16. Device according to one of the claims 1 to 15, **characterized in that** the foil (5) is made of one of polyvinyl chloride, polyethylene and polypropylene.

17. Device according to one of the claims 1 to 15, **characterized in that** the foil (5) is made of medical grade plastic.

## Patentansprüche

1. Schutzvorrichtung für Augenlaserbehandlung, die eine transparente Folie (5) zum Schützen eines Auges (1) gegen direkten Kontakt durch einen Referenzkörper (3), der zwischen einem Laserapplikator (4) und dem Auge (1) angeordnet ist, und Einrichtungen zum Anordnen der Folie (5) zwischen dem Referenzkörper (3) und dem Auge (1) umfasst,
**dadurch gekennzeichnet,**
**dass** die Einrichtungen zum Anordnen der Folie (5) einen Saugring (6) aufweisen, der zum Anbringen an dem Auge (1) um einen Operationsbereich herum ausgelegt ist, dass der Saugring (6) so ausgelegt ist, dass er die Folie (5) so aufnimmt, dass der Operationsbereich von der Folie (5) abgedeckt ist in einem Zustand, in dem der Saugring (6) an dem Auge (1) angebracht ist, und dass der Saugring (6) mit Kopplungseinrichtungen versehen ist zum lösbaren Koppeln des Saugrings (6) mit einem des Referenzkörpers (3) und des Laserapplikators (4) mit dem daran angebrachten Referenzkörper (3) so, dass die Folie (5) zwischen dem Auge (1) und dem Referenzkörper (3) angeordnet ist in dem Zustand, in dem der Saugring (6) an dem Auge (1) angebracht ist und mit dem Referenzkörper (3) oder dem Laserapplikator (4) mit dem daran angebrachten Referenzkörper (3) gekoppelt ist.

2. Vorrichtung nach Anspruch 1, **dadurch gekennzeichnet, dass** die Einrichtungen zum Anordnen der Folie (5) einen Trägerrahmen (51) aufweisen und dass die Folie (5) an dem Trägerrahmen (51) angebracht ist.

3. Vorrichtung nach Anspruch 2, **dadurch gekennzeichnet, dass** der Trägerrahmen (51) mit Kopplungseinrichtungen zum lösbaren Koppeln des Trägerrahmens (51) mit dem Saugring (6) versehen ist.

4. Vorrichtung nach einem der Ansprüche 2 oder 3, **dadurch gekennzeichnet, dass** der Saugring (6) so ausgelegt ist, dass er den Trägerrahmen (51) so aufnimmt, dass der Operationsbereich von der Folie (5) abgedeckt ist in dem Zustand, in dem der Saugring (6) an dem Auge (1) angebracht ist.

5. Vorrichtung nach Anspruch 1, **dadurch gekennzeichnet, dass** die Folie (5) so an dem Saugring (6) angebracht ist, dass der Operationsbereich von der Folie (5) abgedeckt ist in dem Zustand, in dem der Saugring (6) an dem Auge (1) angebracht ist.

6. Vorrichtung nach einem der Ansprüche 1 bis 5, **dadurch gekennzeichnet, dass** der Saugring (6) und die Folie (5) eine Vakuumkammer bilden in dem Zustand, in dem der Saugring (6) an dem Auge (1) angebracht ist.

7. Vorrichtung nach einem der Ansprüche 1 bis 6, **dadurch gekennzeichnet, dass** der Saugring (6) eine erste Öffnung (62) aufweist, die von der Folie (5) verschlossen ist, dass der Saugring (6) eine zweite Öffnung (63) aufweist, die von dem Auge (1) verschlossen ist in dem Zustand, in dem der Saugring (6) an dem Auge (1) angebracht ist, und dass der Operationsbereich mit der Folie (5) in Kontakt steht in dem Zustand, in dem der Saugring (6) an dem Auge (1) angebracht ist.

8. Vorrichtung nach Anspruch 7, **dadurch gekennzeichnet, dass** die erste Öffnung (62) durch einen Vorsprung (61) des Saugrings (6) begrenzt ist, der sich in ein Inneres des Saugrings (6) erstreckt, und dass die erste Öffnung (62) von der Folie (5) verschlossen ist, die auf dem Vorsprung (61) platziert ist.

9. Vorrichtung nach einem der Ansprüche 2 bis 4, **dadurch gekennzeichnet, dass** der Trägerrahmen (51) und die Folie (5) einstückig ausgebildet sind.

10. Vorrichtung nach einem der Ansprüche 1 bis 9, **dadurch gekennzeichnet, dass** die Einrichtungen zum Anordnen der Folie (5) so ausgeführt sind, dass sie die Folie (5) so positionieren, dass ein Spalt zwischen der Folie (5) und dem Referenzkörper (3) vorhanden ist in einem Zustand bevor der Referenzkörper (3) an dem Auge (1) angebracht ist, und so, dass bei einem Prozess, bei dem der Referenzkörper (3) an dem Auge (1) angebracht wird, eine Kontaktregion der Folie (5), die mit dem Referenzkörper (3) in Kontakt kommt, zwischen dem Referenzkörper (3) und dem Auge (1) angeordnet ist und ein Fluid, das sich in einem Bereich zwischen der Kontaktregion und dem Referenzkörper (3) befindet, aus letzterem Bereich herausgepresst wird.

11. Vorrichtung nach einem der Ansprüche 1 bis 10, **dadurch gekennzeichnet, dass** die Folie (5) mit einem spezifizierten Bereich mit einer Undurchsichtigkeit versehen ist, um dadurch einen Operationsbereich des Auges (1) zu begrenzen.

12. Vorrichtung nach einem der Ansprüche 1 bis 11, **dadurch gekennzeichnet, dass** die Folie (5) einen Brechungsindex aufweist, der dem Brechungsindex des Gewebes des Auges (1) und/oder dem Brechungsindex des Referenzkörpers (3) entspricht.

13. Vorrichtung nach einem der Ansprüche 1 bis 12, **dadurch gekennzeichnet, dass** die Folie (5) Flächenstrukturen auf einer Innenfläche der Folie (5) aufweist, die mit dem Auge (1) in Kontakt kommen, um dadurch Fluide von und zu einer Außenfläche des Auges (1), die mit der Folie (5) in Kontakt steht, zu leiten.

14. Vorrichtung nach einem der Ansprüche 1 bis 13, **dadurch gekennzeichnet, dass** die Folie (5) eine elastische Beschichtung auf einer Innenfläche der Folie (5) aufweist, die mit dem Auge (1) in Kontakt kommt, um dadurch unebene Bereiche einer Außenfläche des Auges (1), die mit der Folie (5) in Kontakt steht, auszugleichen.

15. Vorrichtung nach einem der Ansprüche 1 bis 14, **dadurch gekennzeichnet, dass** die Folie (5) Perforationen aufweist, die den Austausch von Fluiden zwischen einer Innenfläche der Folie (5), die mit dem Auge (1) in Kontakt steht, und einer Außenfläche der Folie (5), die mit dem Referenzkörper (3) in Kontakt steht, zu ermöglichen.

16. Vorrichtung nach einem der Ansprüche 1 bis 15, **dadurch gekennzeichnet, dass** die Folie (5) aus einem von Polyvinylchlorid, Polyethylen und Polypropylen gefertigt ist.

17. Vorrichtung nach einem der Ansprüche 1 bis 15, **dadurch gekennzeichnet, dass** die Folie (5) aus einem medizinischen Kunststoff gefertigt ist.

## Revendications

1. Dispositif de protection pour traitement au laser ophtalmique, comprenant une feuille transparente (5) destinée à protéger un oeil (1) d'un contact direct avec un corps de référence (3) disposé entre un applicateur laser (4) et l'oeil (1), et des moyens pour disposer la feuille (5) entre le corps de référence (3) et l'oeil (1), **caractérisé**
**en ce que** les moyens pour disposer la feuille (5) comprennent une bague d'aspiration (6) conçue pour une fixation à l'oeil (1) autour d'une zone opératoire, en ce que la bague d'aspiration (6) est conçue pour recevoir la feuille (5) de telle sorte que la zone opératoire soit recouverte par la feuille (5) dans un état où la bague d'aspiration (6) est fixée à l'oeil (1), et en ce que la bague d'aspiration (6) soit pourvue de moyens de couplage pour coupler de manière amovible la bague d'aspiration (6) à un élément parmi le corps de référence (3) et l'applicateur laser (4) avec le corps de référence (3) étant fixé à celui-ci, de manière à ce que la feuille (5) soit interposée entre l'oeil (1) et le corps de référence (3) dans l'état où la bague d'aspiration (6) est fixée à l'oeil (1), et est couplée au corps de référence (3) ou à l'applicateur laser (4) avec le corps de référence (3) étant fixé à celui-ci.

2. Dispositif selon la revendication 1, **caractérisé en ce que** les moyens pour disposer la feuille (5) comprennent un châssis porteur (51) ; et **en ce que** la feuille (5) est fixée au châssis porteur (51).

3. Dispositif selon la revendication 2, **caractérisé en ce que** le châssis porteur (51) est pourvu de moyens de couplage pour coupler de manière amovible le châssis porteur (51) à la bague d'aspiration (6).

4. Dispositif selon l'une des revendications 2 ou 3, **caractérisé en ce que** la bague d'aspiration (6) est conçue pour recevoir le châssis porteur (51) de telle sorte que la zone opérative soit recouverte par la feuille (5) dans l'état où la bague d'aspiration (6) est fixée à l'oeil (1).

5. Dispositif selon la revendication 1, **caractérisé en ce que** la feuille (5) est fixée à la bague d'aspiration (6) de manière à ce que la zone opérative soit couverte par la feuille (5) dans l'état où la bague d'aspiration (6) est fixée à l'oeil (1).

6. Dispositif selon l'une des revendications 1 à 5, **caractérisé en ce que** la bague d'aspiration (6) et la feuille (5) forment une chambre à vide dans l'état où la bague d'aspiration (6) est fixée à l'oeil (1).

7. Dispositif selon l'une des revendications 1 à 6, **caractérisé en ce que** la bague d'aspiration (6) a une première ouverture (62) fermée par la feuille (5), et **en ce que** la bague d'aspiration (6) a une seconde ouverture (63) fermée par l'oeil (1), dans l'état où la bague d'aspiration (6) est fixée à l'oeil (1), et **en ce que** la zone opérative est en contact avec la feuille (5) dans l'état où la bague d'aspiration (6) est fixée à l'oeil (1).

8. Dispositif selon la revendication 7, **caractérisé en ce que** la première ouverture (62) est délimitée par une saillie (61) de la bague d'aspiration (6) se prolongeant à l'intérieur de la bague d'aspiration (6), et **en ce que** la première ouverture (62) est fermée par la feuille (5) placée sur la saillie (61).

9. Dispositif selon une des revendications 2 à 4, **caractérisé en ce que** le châssis porteur (51) et la feuille (5) sont constitués d'une seule pièce.

10. Dispositif selon l'une des revendications 1 à 9, **caractérisé en ce que** les moyens pour disposer la feuille (5) sont configurés pour positionner la feuille (5) de telle manière qu'il existe un écart entre la feuille (5) et le corps de référence (3) dans un état antérieur à celui où le corps de référence (3) est appliqué sur l'oeil (1), et de telle manière que, dans un processus où le corps de référence (3) est appliquée sur l'oeil (1), une région de contact de la feuille (5) qui vient en contact avec le corps de référence (3) est interposée entre le corps de référence (3) et l'oeil (1), et le fluide qui se situe dans une zone entre la région de contact et le corps de référence (3) est chassé hors de cette dernière zone.

11. Dispositif selon l'une des revendications 1 à 10, **caractérisé en ce que** la feuille (5) est pourvue d'une zone spécifiée de non-transparence afin de limiter ainsi une zone opératoire de l'oeil (1).

12. Dispositif selon l'une des revendications 1 à 11, **caractérisé en ce que** la feuille (5) a un indice de réfraction correspondant à l'indice de réfraction du tissu de l'oeil (1) et/ou à l'indice de réfraction du corps de référence (3).

13. Dispositif selon l'une des revendications 1 à 12, **caractérisé en ce que** la feuille (5) présente des structures de surface sur une surface intérieure de la feuille (5) en contact avec l'oeil (1) pour, par ce moyen conduire les fluides depuis, et vers, une surface extérieure de l'oeil (1) étant en contact avec la feuille (5).

14. Dispositif selon l'une des revendications 1 à 13, **caractérisé en ce que** la feuille (5) a un revêtement élastique sur une surface intérieure de la feuille (5) en contact avec l'oeil (1) pour ainsi égaliser les zones inégales d'une surface extérieure de l'oeil (1) étant en contact avec la feuille (5).

15. Dispositif selon l'une des revendications 1 à 14, **caractérisé en ce que** la feuille (5) a des perforations pour permettre l'échange de fluides entre une surface intérieure de la feuille (5) en contact avec l'oeil (1) et une surface extérieure de la feuille (5) en contact avec le corps de référence (3).

16. Dispositif selon l'une des revendications 1 à 15, **caractérisé en ce que** la feuille (5) est constituée de chlorure de polyvinyle, de polyéthylène et de polypropylène.

17. Dispositif selon l'une des revendications 1 à 15, **caractérisé en ce que** la feuille (5) est constituée d'une matière plastique de qualité médicale.
